# EUROPEAN PATENT SPECIFICATION

(11) **EP 1 589 105 B1**
(45) Date of publication and mention of the grant of the patent: **18.11.2009**
(21) Application number: 05103113.6
(22) Date of filing: 19.04.2005
(51) Int. Cl.: C12N 15/10, C12Q 1/68

(54) **Extraction of nucleic acids using small diameter magnetically-responsive particles**
Extraktion von Nukleinsäuren mittels kleindiametriger magnetischer Partikel
Extraction d'acides nucléiques au moyen de particules magnétiques de faible diamètre

(30) Priority: 23.04.2004 US 564587; 08.07.2004 US 885949
(43) Date of publication of application: 26.10.2005
(73) Proprietor: Becton Dickinson and Company, Franklin Lakes, New Jersey 07417 (US)
(72) Inventor: Fort, Thomas L., Finksburg, Maryland 21048 (US); Collis, Matthew P., Seven Valleys, Pennsylvania 17360 (US)
(74) Representative: Helbing, Jörg

(56) References cited:
- EP-A- 0 818 461
- EP-A- 1 002 860
- EP-A- 1 081 234
- US-A1- 2002 014 443
- US-B1- 6 433 160
- FAGGI E ET AL: "Use of magnetic beads to extract fungal DNA." MYCOSES. JAN 2005, vol. 48, no. 1, January 2005 (2005-01), pages 3-7, XP002331440 ISSN: 0933-7407
- AKUTSU JUN-ICHI ET AL: "Development of an integrated automation system with a magnetic bead-mediated nucleic acid purification device for genetic analysis and gene manipulation" BIOTECHNOLOGY AND BIOENGINEERING, vol. 86, no. 6, 20 June 2004 (2004-06-20), pages 667-671, XP002331441 ISSN: 0006-3592

## Description

The present application claims the benefit of priority under 35 U.S.C. § 119 to Provisional U.S. Patent Application No. 60/564,587 filed April 23, 2004.

### FIELD OF THE INVENTION

The present invention is directed to compositions and methods for extracting substances from a sample. More particularly, the present invention is directed to compositions and methods for extracting nucleic acids from samples via reversible binding with small-diameter magnetically-responsive particles.

### BACKGROUND OF THE INVENTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

The isolation and/or separation of nucleic acids is a necessary task in many diagnostic and biochemical procedures. Known techniques for accomplishing this objective include lysing of biological materials to release the nucleic acids contained therein, followed by removal or separation of at least a portion of the nucleic acid.

The nucleic acid can be separated and/or removed via a number of different techniques. One such technique involves reversibly binding the nucleic acid to magnetic particles. U. S. Patent Nos. 5,973,138 and 6,433,160, the contents of which are incorporated herein by reference in their entirety, are also illustrative of separation techniques utilizing magnetically-responsive particles.

While such techniques are generally effective, there is room for improvement. For example, application of a magnetic field to the above-described magnetic particles can cause the particles to "clump" together. This clumping is believed to be due to the residual magnetic attraction retained by the particles after the magnetic field has been removed. This phenomenon requires the application of a "degaussing" procedure to eliminate clumping of the particles.

### SUMMARY OF THE INVENTION

The present invention provides compositions and techniques that are effective in preventing clumping or aggregation of magnetically-responsive particles, thereby avoiding the necessity of a degaussing procedure.

According to a first aspect, the present invention provides a method for reversibly binding at least one nucleic acid molecule comprising: creating a mixture in a container, the mixture comprising the sample, at least one magnetically-responsive particle, and a remainder; and providing the mixture with a pH of less than about 7.0, thereby altering the surface charge properties of the at least one magnetically-responsive particle. The alteration in the surface charge properties of the at least one magnetically-responsive particle causes the at least one molecule of nucleic acid to become non-specifically bound to the at least one magnetically-responsive particle to form a complex. Preferably, the at least one magnetically-responsive particle is uncoated, untreated, lacks surface modification, and has a diameter of about 0.1µm to about 0.3µm.

According to another aspect, the present invention provides a method for reversibly binding at least one nucleic acid molecule comprising: (i) creating a mixture in a container, the mixture comprising a sample comprising at least one molecule of nucleic acid, at least one magnetically-responsive particle having a diameter of about 0.1 µm to about 0.3µm, and a remainder; (ii) providing the mixture with an acidic pH thereby altering the surface charge properties of the at least one magnetically-responsive particle, wherein the alteration in the surface charge properties of the at least one magnetically-responsive particle causes the at least one molecule of nucleic acid to become non-specifically bound to the at least one magnetically-responsive particle to form a complex; (iii) applying a magnetic field to the complex;(iv) removing the remainder of the mixture from the container while the magnetic field is applied to the complex; (v) eluting the at least one molecule of nucleic acid from the at least one magnetically-responsive particle; and (vi) reapplying the magnetic field to the eluted at least one magnetically-responsive particle, thereby separating the at least one magnetically-responsive particle from the at least one molecule of nucleic acid.

### BRIEF DESCRIPTION OF THE DRAWING

The foregoing and other features, aspects and advantages of the present invention will become apparent from the following description, appended claims and the exemplary embodiments shown in the drawings, which are briefly described below. It should be noted that, unless otherwise specified, like elements have the same reference numbers.

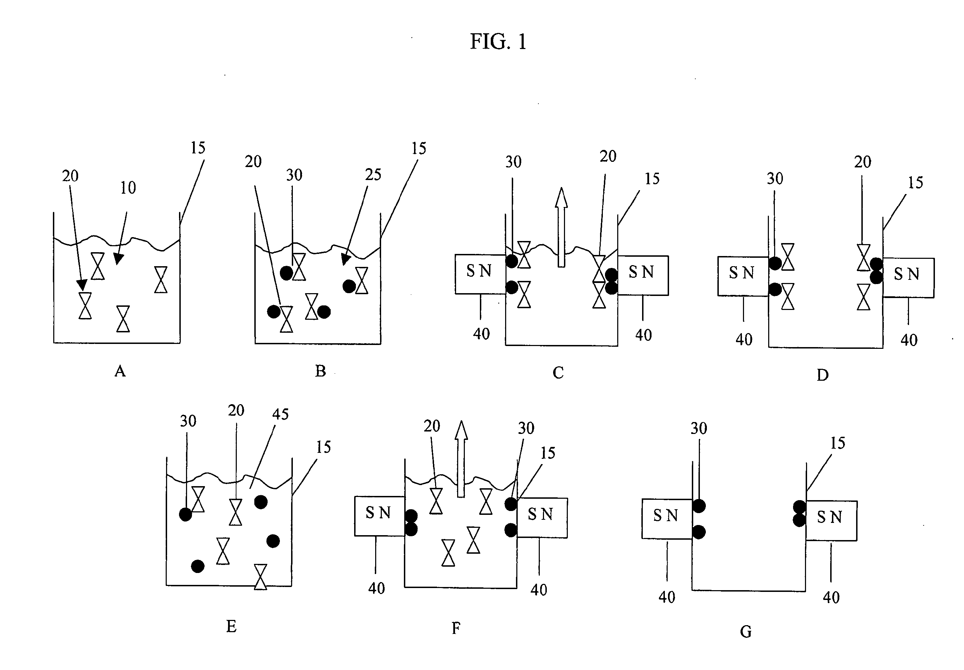

FIG. 1 is a schematic illustration of an embodiment of a process performed according to the principles of the present invention.

### DETAILED DESCRIPTION OF THE INVENTION

The principles of the present invention will now be further described by the following discussion of certain illustrative embodiments thereof and by reference to the foregoing drawing figure.

As used herein, "nucleic acid" means deoxyribonucleic acid (DNA), ribonucleic (RNA) or any naturally occurring or synthetic modification thereof, as well as combinations ofthe foregoing.

As used herein, "sample" means any nucleic acid-containing substance including, but not limited to blood, plasma, serum, urine, bone marrow aspirates, cerebral spinal fluid, tissue, cells, feces, saliva, oral secretions, nasal secretions, bronchial lavage, hair, cervical fluids, vaginal fluids, semen, sputa and lymphatic fluids.

As used herein, "magnetically-responsive particle" means a particle capable of having a magnetic moment imparted thereto, or otherwise moveable under the action of a magnetic field. Such particles include, but are not limited to, ferric hydroxide, ferrosoferric oxide, iron sulfide and iron chloride.

As used herein, "non-specifically bound" means the binding mechanism does not occur via a receptor, capture agent, or the like, which would selectively couple only with a specific agents.

The magnetically-responsive particles of the present invention may be uncoated, untreated, and/or lack any type of surface modification. The magnetically-responsive particles of the present invention bind non-specifically to the nucleic acid, i.e., are non-specifically bound thereto under certain conditions.

The Applicant has found that when in an acidic environment, magnetically-responsive particles will reversibly bind to nucleic acids. Although not desiring to be bound by a particular theory, the Applicant believes that an acidic environment increases the electropositive nature of the particles, thereby increasing the binding of the particles to the electronegative nucleic acids.

As noted above, according to another embodiment, the magnetically-responsive particles of the present invention are preferably uncoated, untreated and/or lack surface modification. The particles, therefore, bind non-specifically to the nucleic acids. Thus, iron particles are useful in the present invention, and the iron may be an iron oxide of forms such as ferric hydroxide and ferrosoferric oxide, which have low solubility in an aqueous environment. Other iron particles such as iron sulfide and iron chloride may also be suitable for binding and extracting nucleic acids using the conditions described herein.

The shape of the magnetically-responsive particles is not critical to the present invention. The magnetically-responsive particles may be of various shapes including, for example, spheres, cubes, oval, capsule-shaped, tablet-shaped, nondescript random shapes, etc., and may be of uniform shape or non-uniform shape. Whatever the shape of the particle, its average diameter at its widest point is generally from about 0.1 µm to about 0.3µm. According to one embodiment, the magnetically-responsive particles have a diameter of about 0.3µm.

The acidic environment in which the magnetically-responsive particles effectively and reversibly bind to nucleic acid can be provided through a variety of means. For example, the magnetically-responsive particles can be added to an acidic solution, or an acidic solution may be added to the particles. Alternatively, a solution or environment in which the magnetically-responsive particles are located can be acidified by addition of an acidifying agent such as hydrochloric acid, sulfuric acid, acetic acid or citric acid. Provided that the environment in which the magnetically-responsive particles are located is of a pH less than about 7.0, the particles will reversibly bind nucleic acids, as well as whole, intact particles or organisms.

The bound nucleic acid/magnetic particle complex can be eluted into an appropriate buffer for further manipulation. Heating the environment of the particles with bound nucleic acid and/or raising the pH of such environment can accomplish such elution. Agents that can be used to aid the elution of nucleic acid from magnetically-responsive particles include basic solutions or buffers such as potassium hydroxide, sodium hydroxide or any compound that will increase the pH of the environment to an extent sufficient that electronegative nucleic acid is displaced from the magnetically-responsive particles.

The nucleic acid can then be extracted, concentrated and/or isolated. Subsequently, the nucleic acid can be subjected to further processes, such as one or more of: cultivation, polymerase chain amplification, reverse transcription polymerase chain reaction, strand displacement amplification, reverse transcriptase strand displacement amplification, cloning, sequencing, transcription mediated amplification and ligase chain amplification.

An exemplary process performed according to the principles of the present invention will l now be described by reference to FIG. 1. In step A, a sample 10 is located in a container 15. The sample 10 contains nucleic acid 20.

A mixture 25 is then formed in step B that includes the sample 10, nucleic acid 20 and magnetically-responsive particles 30. As noted above, the magnetically-responsive particles 30 can have a diameter of about 0.1 µm to about 0.3µm, and may be about 0.3 µm. The particles 30 can be uncoated, untreated and lack surface modification. The pH of this mixture is brought to an appropriate level, preferably to below about 7.0 or below. The mixture can be formed by any suitable means. For example, the magnetically-responsive particles 30 can be added to an acidic solution, or an acidic solution may be added to the particles 30. Alternatively, a solution or environment in which the magnetically-responsive particles 30 are located can be acidified by addition of an acidifying agent such as hydrochloric acid, sulfuric acid, acetic acid or citric acid.

An extraction control solution may optionally be added to the above-described mixture. The extraction control solution contains an agent that can be detected or observed, the presence of which being indicative of either a successful or unsuccessful extraction procedure. The extraction control solution can have any suitable composition and contain any suitable agent. By way of example, the extraction control solution may contain an agent in the form of an oligonucleotide with a fluorophore linked thereto. The agent also binds to the magnetically-responsive particles. The presence of the agent can then be detected in a subsequent amplification assay, thereby indicating whether a desired extraction has been successful.

As previously described, the change in pH causes a modification of the surface charge characteristics of at least the magnetically-responsive particles 30, causing the nucleic acid 20 to become bound to the magnetically-responsive particles 30, thereby forming a complex. A magnetic field is then applied. As illustrated in step C, this can be accomplished by bringing opposing permanent magnets 40, or electromagnets (not shown), into close proximity with the outside of the container 15. Under the influence of the magnetic field, the bound nucleic acid 20/magnetically-responsive particle 30 complex is drawn toward the magnets 40. The supernatant, or remainder, of the mixture 25 can then be removed from the container 15 (step D).

One or more washing steps (not shown) may optionally be performed at this stage to further eliminate undesirable substances. Any suitable wash may be utilized. For example, water, a non-ionic detergent or a non-ionic detergent/low concentration acid solution may be utilized.

Step E is illustrative of eluting the complex to free the nucleic acid 20 from the magnetic particles. This elution can be accomplished by any suitable means such as by chemical agent, thermal energy, or a combination of the two. For example, a buffer agent 45 can be added to increase the pH to a suitable level. According to one embodiment, the pH is raised to approximately 8.3-8.4. The buffer may comprise KOH.

The magnets 40 are then brought back into close proximity with the container 15 in step F, which now draws just the magnetically-responsive particles 30 to the sidewalls of the container 15. The nucleic acid 20 can then be removed from the container 15 (step G).

An advantage of the use of the above-described magnetically-responsive particles of the present invention is that the above-described separation/extraction method can be accomplished without a "degaussing" step, which is often required in conventional techniques to remove residual magnetic attraction between particles and which can cause "clumping."

Subsequent to step G, the nucleic acid 20 can be subjected to further processes, such as one or more of the following: cultivation, polymerase chain amplification, reverse transcription polymerase chain amplification, strand displacement amplification, reverse transcriptase strand displacement amplification, and ligase chain amplification.

The above-described steps of the exemplary process may be carried out manually, in automated fashion, or by a combination of manual and automated steps. The automated steps may be performed with an automated robotic device, which optionally includes automated pipetting, mixing, and magnet positioning functionality. The automated robotic device may be computer controlled. One such device is commercially available from Becton, Dickinson and Company as the BD Viper™.

The present invention can be used in a number of different contexts. For example, the present invention may be utilized in connection with systems and methods of the type described in U.S. Patent No. 6,672,458, the content of which is incorporated herein by reference in its entirety. These types of systems and methods generally involve the extraction, amplification and detection of target species present in a biological sample. Such a process may involve any suitable combination of the following steps.

A container in the form of an extraction tube is provided with magnetically-responsive particles. A lysing agent is dispensed into the extraction tube containing the magnetically responsive particles by an automated robotic device.

A sample is then dispensed into the extraction tube, also by the automated robotic device, and then mixed to lyse the organism(s) of interest contained in the sample at a high pH, thereby releasing nucleic acid. An acid solution may be added to the extraction tube. The acid solution acts to normalize different types of samples, thereby promoting a consistent pH.

An extraction control solution can be added to the extraction tubes by the automated robotic device, and mixed. The extraction control solution contains an oligonucleotide and fluorophore linked thereto that, along with the nucleic acid, binds to the magnetically-responsive particles to form a complex. The fluorophore linked to the oligonucleotide is carried through the extraction, and the amount of fluorophore is read in a subsequent amplification assay and indicates a successful or unsuccessful extraction. A magnetic field is applied to the contents of the extraction tube by bringing a pair of opposing magnets into close proximity with the outside of the tube, thereby drawing the complex to the inner periphery of the tube. The automated robotic device then aspirates the contents of the tube, leaving the complex therein.

The complex is then washed with a solution of, for example, non-ionic detergent. One suitable non-ionic detergent is commercially available as "Tween® 20." Residual acid contained in the interstitial spaces between the magnetically-responsive particles maintains an acidic pH. Alternatively, a mild acidic solution may be added to the non-ionic detergent. After washing, the magnetic field is reapplied to draw the complex to the inner periphery of the tube, and the wash is aspirated out of the tube.

An elution buffer is then added to the extraction tube, and mixed, to elute the nucleic acid from the complex. The elution buffer is added and mixed by the automated robotic device. The nucleic acid can then be separated from the magnetically-responsive particles by manipulation of the magnets in the manner described above.

Subsequent to the above-described extraction, the nucleic acid can be subjected to additional processes, such as techniques to detect and/or quantify target analytes. For example, any suitable method of amplification may be used in the methods of the invention. Such methods include, but are not limited to, polymerase chain reaction ("PCR"), Strand Displacement Amplification ("SDA"), thermophilic Strand Displacement Amplification ("tSDA"), Self-Sustained Sequence Replication ("3SR"), Nucleic Acid Sequence-Based Amplification ("NASBA"), Qβ replicase systems; Ligase Chain Reaction ("LCR"), and transcription-mediated amplification ("TMA"). Detection can be accomplished by any suitable mechanism. For example, the increase in fluorescence of a reporter probe.

Specific non-limiting examples comprehended by the principles of the present invention will now be described to further illustrate the concepts of the present invention.

### Example 1

An experiment was performed to compare the performance of various amounts of small-diameter magnetic particles (0.3 µm) with a standard amount of larger diameter particles (1.0 µm) within the context of an assay for *Chlamydia trachomatis* (CT).

Containers in the form of extraction tubes are provided with magnetically-responsive particles. The small diameter magnetic particles are identified as ferrosoferric oxide TB 5600, commercially available from Elementis. These particles have an average diameter of about 0.3 µm. The larger diameter magnetic particles are identified as ferrosoferric oxide M-25 RPS, commercially available from PEA Ridge. These particles have an average diameter of about 1.0 µm. The particles were added to the tubes according to the following schedule.

**Table I**

| Tube no. | Average Particle Size | Amount |
|---|---|---|
| 1 | 0.3 µm | 2.5 mg |
| 2 | 0.3 µm | 5.0 mg |
| 3 | 0.3 µm | 10.0 mg |
| 4 | 0.3 µm | 20.0 mg |
| 5 | 0.3 µm | 40.0 mg |
| 6 | 1.0 µm | 40.0 mg |

The particles were gravimetrically measured and hand dispensed into extraction tubes.

A solution of 5M strength phosphoric acid (H₃PO₄) is dispensed into each extraction tube containing the magnetically-responsive particles by the BD Viper™ device.

Urine samples were spiked at 100 particles/ml. An automated robotic device dispensed 950 µL of the spiked urine samples into extraction tubes. The robotic device dispensed 50 µL of 5M H₃PO₄ into extraction tubes. The urine samples are mixed with the phosphoric acid solution to lyse the organism(s) of interest contained in the sample, thereby releasing nucleic acid. The electronegative nucleic acid released from a cell is associated with the electropositive particles.

A magnetic field is applied to the contents of the extraction tube. The automated robotic device then aspirates the contents of the tube, leaving the complex therein, and the magnetic field is removed from the container. The automated robotic device performs a double extraction by repeating the above-described steps beginning with the introduction of spiked urine into the extraction tube.

The complex is then washed with a 1mM solution of Glycine-HCl wash. After washing, the magnetic field is reapplied to draw the complex to the inner periphery of the tube, and the wash is aspirated out of the tube.

An elution buffer solution is then added to the extraction tube, and mixed, to elute the nucleic acid from the particles present in the complex. Approximately 370 µL of elution buffer solution was added to each tube. The elution buffer solution comprises a solution based on a mixture of KOH and Bicine. The elution buffer is added and mixed by the automated robotic device.

The eluted sample containing nucleic acid is transferred to commercially available Chlamydia BDProbeTec™ priming wells at 150 µL well. The priming wells are maintained at room temperature for 20 minutes. The priming wells are transferred to a 72°C heat plate. The Chlamydia ProbeTec amplification wells are transferred to a 54°C heat plate at the same time the priming wells are transferred to the 72°C heat plate. The plates are incubated at temperature for 10 minutes. Following the 10 minute incubation period, 100 µL of solution is transferred from the priming wells to the amplification wells. The solution is mixed three times. An adhesive cover is applied to the top of the wells to prevent evaporative loss of sample during amplification. The plates containing the amplification wells are placed in a BDProbeTec™ reader.

The amplification process was monitored with a BDProbeTec™ reader, which detected the fluorescent increase associated with reporter probe conversion. The reader produces "MOTA" (Measure Other Than Acceleration) values based on the detection of reporter probe conversion during the amplification process. The average MOTA values generated during the monitoring period for each of the above-described samples are reported below in Table II.

**Table II**

| Tube no. | Average MOTA Value |
|---|---|
| 1 | 24,160 |
| 2 | 68,410 |
| 3 | 42,789 |
| 4 | 38,816 |
| 5 | 28,053 |
| 6 | 45,358 |

From the MOTA values reported in Table II, it is evident that the extraction and amplification of the target CT analyte using 5.0 mg of 0.3 µm magnetically-responsive particles (tube no. 2.) was more successful than the extraction and amplification using 40.0 mg of 1.0 µm magnetic particles (tube no. 6), as evidenced by the higher average MOTA value associated with tube no. 2. In the case of the run using only 2.5 mg of the 0.3 µm particles (tube no. 1), it is believed that the lower average MOTA value is attributable to a deficiency in binding area ofthe particles and/or a high percentage of the 0.3 µm sticking to the pipette tip during dispensing. In the case of those runs utilizing more than 5.0 mg of the 0.3 µm particles (tube nos. 3, 4, 5), the lower average MOTA values are attributable to increased inhibition in the amplification procedure due to the higher the mass of iron, and the more interstitial space between particles, which tends to increase the amount of potential amplification inhibitors and other urine specific negatively charged components present during amplification.

### Example 2

An experiment was performed to determine optimal quantities of magnetic particles and phosphoric acid lysing agent to use in the extraction of a target analyte.

Containers in the form of extraction tubes are provided with magnetically-responsive particles. The magnetically-responsive particles are the ferrosoferric oxide Elementis TB 5600 particles mentioned in Example 1.

A solution of 5M strength phosphoric acid (H₃PO₄) is dispensed into each extraction tube containing the magnetically-responsive particles. The phosphoric acid was dispensed by an automated robotic device, namely the BD VIPER™ automated extractor device.

The extraction tubes were loaded with magnetic particles and phosphoric acid to the following schedule set forth in Table III.

**Table III**

| Tube no. 1 | Mass of Iron Particles | Volume of Phosphoric Acid |
|---|---|---|
| 1 | 5 mg | 50 ml |
| 2 | 5 mg | 80 ml |
| 3 | 5 mg | 110 ml |
| 4 | 5 mg | 140 ml |
| 5 | 10 mg | 50 ml |
| 6 | 10 mg | 80 ml |
| 7 | 10 mg | 110 ml |
| 8 | 10 mg | 140 ml |
| 9 | 20 mg | 50 ml |
| 10 | 20 mg | 80 ml |
| 11 | 20 mg | 110 ml |
| 12 | 20 mg | 140 ml |

Urine samples having a volume of 1.0 ml were then dispensed into the extraction tubes, also by the automated robotic device. The urine samples are spiked to a level of 100 particles/ml, and mixed with the phosphoric acid solution to lyse the organism(s) of interest contained in the sample, thereby releasing nucleic acid.

A magnetic field is applied to the contents of the extraction tube. The automated robotic device brings a pair of opposing magnets into close proximity with the outside of the tube, thereby drawing the complex to the inner periphery of the tube. The automated robotic device then aspirates the contents of the tube, leaving the complex therein, and the magnetic field is removed from the container.

The complex is then washed with a 1mM solution of Glycine-HCl wash by the automated robotic device. After washing, the magnetic field is reapplied to draw the complex to the inner periphery of the tube, and the wash is aspirated out of the tube.

An elution buffer solution was then added to the extraction tube by the BD Viper™, and mixed, to elute the nucleic acid from the complex. A 370µL quantity of buffer was added to each tube. The elution buffer solution comprises a solution based on a mixture of KOH and Bicine. The eluted sample nucleic acid is then added to priming wells subjected to the same strand displacement amplification process described in Example 1. The target analyte was CT.

For the above-described assay, the following data contained in Table IV was collected.

**Table IV**

| **Tube No.** | **Average MOTA value** |
|---|---|
| 1 | 77,391 |
| 2 | 82,371 |
| 3 | 60,109 |
| 4 | 53,540 |
| 5 | 84,785 |
| 6 | 76,455 |
| 7 | 72,180 |
| 8 | 77,665 |
| 9 | 42,113 |
| 10 | 50,654 |
| 11 | 52,405 |
| 12 | 48,319 |

From the above data it is evident that those tubes containing 5 mg and 10 mg of the particles exhibited the best performance. Tube nos. 2, 5 and 8 (5 mg/80 ml, 10 mg/50 ml and 10 mg/140 ml, respectively) exhibited the best performance, as exhibited by the highest average MOTA values.

While this invention is satisfied by embodiments in many different forms, as described in detail in connection with preferred embodiments of the invention, it is understood that the present disclosure is to be considered as exemplary of the principles ofthe invention and is not intended to limit the invention to the specific embodiments illustrated and described herein. Numerous variations may be made by persons skilled in the art without departure from the spirit of the invention.

## Claims

1. A method for reversibly binding at least one nucleic acid molecule comprising:
(i) creating a mixture in a container, the mixture comprising a sample comprising at least one molecule of nucleic acid, at least one magnetically-responsive particle having a diameter of about 0.1 µm to about 0.3µm, and a remainder; and
(ii) providing the mixture with an acidic pH thereby altering the surface charge properties of the at least one magnetically-responsive particle;
wherein the alteration in the surface charge properties of the at least one magnetically-responsive particle is such that the at least one molecule of nucleic acid to become non-specifically bound to the at least one magnetically-responsive particle to form a complex.

2. The method of claim 1, wherein the at least one magnetically-responsive particle is uncoated, untreated, and lacks surface modification.

3. The method of claim 1, wherein step (i) comprises adding the at least one magnetically-responsive particle to the container, adding a lysing agent to the container, adding the sample to the container, and mixing.

4. The method of claim 1, wherein the at least one magnetically-responsive particle comprises an iron oxide particle.

5. The method of claim 1, wherein the at least one magnetically-responsive particle has a diameter of about 0.3µm.

6. The method of claim 1, further comprising the step of applying a magnetic field to the complex.

7. The method of claim 6, further comprising the additional step of eluting the at least one molecule of nucleic acid from the at least one magnetically-responsive particle.

8. The method of claim 7, wherein the step of eluting comprises adding a buffer solution to the container.

9. The method of claim 8, further comprising the step of reapplying a magnetic field to the eluted at least one magnetically-responsive particle, thereby separating the at least one magnetically-responsive particle from the at least one molecule of nucleic acid.

10. The method of claim 9, further comprising at least one of PCR, SDA, tSDA, 3SR, NASBA, Qβ, LCR and TMA.

## Patentansprüche

1. Verfahren zum reversiblen Binden wenigstens eines Nucleinsäuremoleküls, umfassend:
(i) Bereitstellen eines Gemischs in einem Behälter, wobei das Gemisch eine Probe umfasst, die wenigstens ein Nucleinsäuremolekül, wenigstens ein magnetisch ansprechbares Teilchen mit einem Durchmesser von etwa 0,1 µm bis etwa 0,3 µm und einen Rest umfasst; und
(ii) Versehen des Gemischs mit einem sauren pH-Wert, wodurch die Oberflächenladungseigenschaften des wenigstens einen magnetisch ansprechbaren Teilchens verändert werden;
wobei die Veränderung der Oberflächenladungseigenschaften des wenigstens einen magnetisch ansprechbaren Teilchens derart sind, dass das wenigstens eine Nucleinsäuremolekül unter Bildung eines Komplexes unspezifisch an das wenigstens eine magnetisch ansprechbare Teilchen gebunden wird.

2. Verfahren gemäß Anspruch 1, wobei das wenigstens eine magnetisch ansprechbare Teilchen unbeschichtet und unbehandelt ist und keine Oberflächenmodifikation aufweist.

3. Verfahren gemäß Anspruch 1, wobei Schritt (i) das Hinzufügen des wenigstens einen magnetisch ansprechbaren Teilchens in den Behälter, das Hinzufügen eines Lysemittels in den Behälter, das Hinzufügen der Probe in den Behälter und Mischen umfasst.

4. Verfahren gemäß Anspruch 1, wobei das wenigstens eine magnetisch ansprechbare Teilchen ein Eisenoxidteilchen umfasst.

5. Verfahren gemäß Anspruch 1, wobei das wenigstens eine magnetisch ansprechbare Teilchen einen Durchmesser von etwa 0,3 µm hat.

6. Verfahren gemäß Anspruch 1, das weiterhin den Schritt des Anlegens eines Magnetfelds an den Komplex umfasst.

7. Verfahren gemäß Anspruch 6, das weiterhin den zusätzlichen Schritt des Ablösens des wenigstens einen Nucleinsäuremoleküls von dem wenigstens einen magnetisch ansprechbaren Teilchen umfasst.

8. Verfahren gemäß Anspruch 7, wobei der Schritt des Ablösens das Hinzufügen einer Pufferlösung in den Behälter umfasst.

9. Verfahren gemäß Anspruch 8, das weiterhin den Schritt des erneuten Anlegens eines Magnetfelds an das abgelöste, wenigstens eine magnetisch ansprechbare Teilchen umfasst, wodurch das wenigstens eine magnetisch ansprechbare Teilchen von dem wenigstens einen Nucleinsäuremolekül getrennt wird.

10. Verfahren gemäß Anspruch 9, das weiterhin wenigstens eines der Verfahren PCR, SDA, tSDA, 3SR, NASBA, Qβ, LCR und TMA umfasst.

## Revendications

1. Procédé pour lier de façon réversible au moins une molécule d'acide nucléique comprenant :
(i) la préparation d'un mélange dans un récipient, le mélange comprenant un échantillon comprenant au moins une molécule d'acide nucléique, au moins une particule sensible au champ magnétique dont le diamètre est compris entre environ 0,1 µm et environ 0,3 µm, et un restant ; et
(ii) l'ajustement du mélange à un pH acide, modifiant ce faisant les propriétés de charge superficielle du ou des particules sensibles au champ magnétiques ;
dans lequel la modification des propriétés de charge superficielle du ou des particules sensibles au champ magnétique est telle que la ou les molécules d'acide nucléique deviennent liées de façon non spécifique à la ou les particules sensibles au champ magnétique pour former un complexe.

2. Procédé selon la revendication 1, dans lequel la ou les particules sensibles au champ magnétique sont non enrobées, non traitées et exemptes de modifications de surface.

3. Procédé selon la revendication 1, dans lequel l'étape (i) comprend l'ajout de la ou les particules sensibles au champ magnétique dans le récipient, l'ajout d'un agent de lyse dans le récipient, l'ajout de l'échantillon dans le récipient, et le mélange.

4. Procédé selon la revendication 1, dans lequel la ou les particules sensibles au champ magnétique comprennent une particule d'oxyde de fer.

5. Procédé selon la revendication 1, dans lequel la ou les particules sensibles au champ magnétique ont un diamètre de l'ordre de 0,3 µm.

6. Procédé selon la revendication 1, comprenant en outre l'étape consistant à appliquer un champ magnétique au complexe.

7. Procédé selon la revendication 6, comprenant en outre l'étape supplémentaire consistant à éluer la ou les molécules d'acide nucléique à partir de la ou les particules sensibles au champ magnétique.

8. Procédé selon la revendication 7, dans lequel l'étape d'élution comprend l'ajout d'une solution tampon dans le récipient.

9. Procédé selon la revendication 8, comprenant en outre l'étape consistant à ré-appliquer un champ magnétique à la ou les particules sensibles au champ magnétique éluée, séparant ce faisant la ou les particules sensibles au champ magnétique de la ou des molécules d'acide nucléique.

10. Procédé selon la revendication 9, comprenant en outre au moins un élément parmi PCR, SDA, tSDA, 3SR, NASBA, Qβ, LCR et TMA.
